# EUROPEAN PATENT SPECIFICATION

(11) **EP 3 806 777 B1**
(45) Date of publication and mention of the grant of the patent: **08.09.2021**
(21) Application number: 19752523.1
(22) Date of filing: 14.08.2019
(51) Int. Cl.: A61F 2/16

(54) **POSTERIOR CHAMBER PHAKIC INTRAOCULAR LENS**
PHAKISCHE INTRAOKULARLINSE FÜR HINTERE AUGENKAMMER
LENTILLE INTRAOCULAIRE PHAQUE DE CHAMBRE POSTÉRIEURE

(30) Priority: 14.08.2018 BE 201805569; 14.08.2018 US 201816103722
(43) Date of publication of application: 21.04.2021
(73) Proprietor: Physiol, 4031 Angleur (BE)
(72) Inventor: ZALDIVAR, Roger, Mendoza, 5505 (AR); PAGNOULLE, Christophe, 4800 VERVIERS (BE); REDZOVIC, Suad, 4020 JUPILLE SUR MEUSE (BE)
(74) Representative: Gevers Patents
(86) International application number: PCT/EP2019/071831
(87) International publication number: WO 2020/035534

(56) References cited:
- WO-A1-2016/054624
- WO-A1-2016/191614
- US-A1- 2005 021 140
- US-A1- 2005 096 741
- US-A1- 2006 259 140
- US-A1- 2007 168 028

## Description

### Technical field

The present invention concerns an intraocular lens. More specifically, the present invention concerns a posterior chamber phakic intraocular lens.

### Prior art

Generally speaking, phakic intraocular lenses are intraocular lenses intended to be placed in an eye in order to correct defects of vision. These intraocular lenses are generally implanted in patients who are still young, as a complement to the lens of the eye.

There are several classes of phakic intraocular lenses, among which those of posterior chamber phakic intraocular lenses intended to be implanted in an area of the eye between a posterior surface of the iris and an anterior surface of the lens, and at least partially in the ciliary sulcus of the eye.

A defect in implantation of such an intraocular lens lies in the fact that it is likely to be positioned differently from one eye to the other on the basis of some parameters, especially on the basis of the anatomy of the ciliary sulcus, the size of which usually varies by several millimeters from one patient to another. In particular, implantation of a posterior chamber phakic intraocular lens, the size of which may not be adapted, would risk leading to complications that would be more or less serious for a patient, such as:
- for example, in a case where the phakic intraocular lens is too small with respect to the size of the patient's ciliary sulcus: contact with the lens of the eye that has an impact on precision of vision or generates a cataract of the eye, or a loss in the corrective power of the phakic intraocular lens;
- for example, in a case where the phakic intraocular lens is too large with respect to the size of the patient's ciliary sulcus: an inflammation, depigmentation of the iris, pupillary blockage, glaucoma, or a depression between the anterior and posterior chambers of the eye after a blockage of a liquid passing between these chambers.

Production and use of several sizes of posterior chamber phakic intraocular lenses on the basis of the anatomy of the patient's eye cannot completely circumvent this defect. Indeed, shortcomings in terms of the stability of the position of such a phakic intraocular lens once it is implanted in an eye are likely to lead to the same medical complications.

As a first attempt to solve this problem, it was disclosed in document WO 2016/054624 A1 a phakic intraocular lens with a particular haptic structure comprising:
- a proximal haptic part surrounding an optic, and provided with an elevated collar,
- self-adjusting struts spaced from and coupled with lateral wings to the proximal haptic part.

The stability in implantation position in an eye for such phakic intraocular lens can nevertheless be improved.

### Summary of the invention

An object of the present invention is to provide a posterior chamber phakic intraocular lens both adapted to any eye anatomy and particularly stable in implantation position in an eye.

For this purpose, the invention provides a posterior chamber phakic intraocular lens comprising:
- an anterior surface and a posterior surface;
- a central optical part comprising a lens, and extending radially relative to an optical axis directed from the anterior surface to the posterior surface;
- a peripheral haptic part extending radially outward relative to the central optic part;
   the peripheral haptic part comprising:
      - a proximal portion fixed on the central optical part and extending circumferentially and (substantially) symmetrically around the central optical part;
      - a distal portion fixed on the proximal portion and comprising a plurality of support elements extending both radially outward and posteriorly relative to the central optical part;
      the support elements being configured for supporting the phakic intraocular lens on a ciliary zonule when it is in normal use in an eye;
   the central optical part and the peripheral haptic part forming a (rigid) dome;
   - a plurality of elongated flexible haptics,
      each of the flexible haptics extending both partially circumferentially around one of the support elements, and radially outward relative to the central optical part;
      a first diameter of the phakic intraocular lens being strictly larger than a second diameter of an optical assembly consisting in the central optical part and the peripheral haptic part; each of the flexible haptics extending at least partially between the first and second diameters;
      each of the flexible haptics comprising:
         - a proximal extremity mounted on the proximal portion of the peripheral haptic part;
         - a free distal extremity for hooking the phakic intraocular lens into a ciliary sulcus when it is in normal use in an eye.

The posterior chamber phakic intraocular lens provided by the present invention comprises a central optical part equipped with two distinct and complementary haptic structures: the peripheral haptic part comprising the plurality of support elements, and the plurality of flexible haptics. The technical effect of the invention lies mainly in the combination of a structure in the form of a (rigid) dome of the optical assembly, the support elements of which are feet of the dome, supporting the phakic intraocular lens, more specifically the optical assembly, on the ciliary zonule, and the capacity of the flexible haptics to hook into an arbitrary ciliary sulcus given that they extend radially beyond the optical assembly, and that the first diameter is strictly larger than the second diameter. These two haptic structures allow improved stabilisation, both axial and radial as well as in rotation, of the phakic intraocular lens in a implantation site defined in a posterior chamber of an eye, as well as adaptability of the phakic intraocular lens to a broad range of patient eye anatomies.

More specifically, the peripheral haptic part allows stabilisation of the implant in a direction parallel to the optical axis. The support elements are in secant planes with respect to a plane tangent to the central optical part, in a way to support the dome on the ciliary zonule in implantation position. The dome is configured to be anteriorly above a lens of an eye when the phakic intraocular lens is in normal use in the eye, so that it encloses the lens at least anteriorly. As a consequence, the distance, called "the vault", measured along the optical axis, between the lens and the posterior surface of the phakic intraocular lens, is defined and stabilized. It can be assimilated to a safety distance required in order to avoid a contact or too much proximity between the phakic intraocular lens and the lens, which would be likely to lead to physical trauma and medical complications. The distance between the phakic intraocular lens and the iris is also defined and stabilized as being the distance between the anterior surface of the phakic intraocular lens, in other words, the top of the dome, and the posterior surface of the iris.

Preferentially, this distance, called "the vault", is comprised and/or is adjustable between 100 and 1000 microns, more preferably between 350 and 700 microns, with or without radial and/or axial compression. This distance advantageously makes it possible to guarantee enough space between the phakic intraocular lens and both the lens and the iris, and to compensate for an anatomical size defect in the posterior chamber of the eye, or a possible positioning defect of the phakic intraocular lens, to sharply reduce the risks of complication for the patient. According to an embodiment of the invention, this distance is titrated by sculpting the posterior surface of the phakic intraocular lens such that it follows a contour of a specific natural crystalline lens for which it is intended to be implanted.

The structure of the rigid dome of the optical assembly is designed to adapt to any eye on the basis of the dome curvature, the posterior surface of which is preferentially more curved than the anterior surface of any lens, and on the basis of the choice of the second diameter, preferentially between 11 and 12 mm, more preferably with a value of 11.25 mm to be compatible with a standard posterior chamber anatomy of an eye. So the rigid dome structure induced by the optical assembly is above the lens while being appropriately implanted in the posterior chamber of the eye, resting on the ciliary zonule, and stabilizing the phakic intraocular lens parallel to the optical axis. The elongated flexible haptics extend further radially than the optical assembly in the posterior chamber of the eye, and hook into the ciliary sulcus. The flexibility and the geometry of these haptics makes it possible to compensate for the size variations of the ciliary sulcus, so that the phakic intraocular lens in implantation position adapts itself perfectly to the size of the ciliary sulcus, across a range of internal measurements including those which can not be accurately estimated preoperatively, thereby potentially reducing the size of the first diameter of the phakic intraocular lens when it is in normal use in the eye, namely when it is in an implantation position in an eye, while maintaining a dome-shaped optical assembly with a dimension corresponding substantially to the second diameter. Advantageously, production and handling of a single phakic intraocular lens model according to the invention are enough for a broad range of patient eye anatomies.

Moreover, the flexible haptics enable the stabilization of the phakic intraocular lens according to the invention in rotation in a plane perpendicular to the optical axis such that the variation in the internal sulcus diameter (acknowledged to be greater in one orientation than another) can be fully compensated. These flexible haptics are radially and circumferentially extended to also hook into the ciliary sulcus of the eye, playing a role of circumferential anchors for the phakic intraocular lens, like a plane elastic fabric mounted laterally using lateral hooks. These flexible haptics are important in the particular embodiment where the lens is that of a toroidal implant comprising a curvature irregularity to correct astigmatism. In this case, the stability of the angular position of the lens in the perpendicular plane is crucial in order to guarantee the expected optical results. The flexible haptics also contribute to stabilizing the phakic intraocular lens in the aforementioned perpendicular plane when it is in normal use, as well as avoiding possible decentering of the phakic intraocular lens with respect to the optical axis of the eye, which could affect the expected optical results. The flexible haptics work together to maintain the lens in a central optical zone.

The peripheral haptic part and the flexible haptics are preferentially made of a very flexible and very resistant material. They constitute a compromise between the need for stability and compliance with intraocular structures, and the need for rigidity, avoiding excessive exertion of force and trauma to delicate intraocular structures, many of which are complex and not visible during or prior to implantation. The "flexible" characteristic of the flexible haptics is preferentially induced by this material combined with their substantially elongated shape along a curve and/or their low average thickness measured along the optical axis. The "rigid" characteristic of the dome is preferably induced, on the one hand, by a thickness measured in parallel to the optical axis, significantly larger on average, of the peripheral haptic part than that of the flexible haptics, and/or on the other hand, of a flared and/or wide and/or thick shape of the support elements, and/or yet again on the other hand, of the rigidity and adaptability of the geometric technical characteristics of the dome to a broad range of patient eye anatomies. Preferentially, the use of the term "rigid" is less for the purpose of qualifying the dome than to compare it to the flexibles haptiques.

Preferably, the thickness of the peripheral haptic part is targeted to allow the phakic intraocular lens to reside at a particular distance from the anterior surface of a natural crystalline lens by virtue of selectively titrating the curvature of the anterior and/or the posterior surfaces such that the posterior surface mimics the curvature of the specific natural lens.

In the framework of the present document, the terms "first", "second", "third" and "fourth" are solely used to differentiate the various elements and do not imply any order in these elements.

According to a preferred embodiment of the invention:
- the first diameter is comprised between 14 and 15 mm; and/or
- the second diameter is comprised between 11 and 12 mm.

Preferentially, the first diameter is comprised between 14.5 and 15 mm, more preferably equivalent to 14.7 mm, when no radial and/or axial compression force is exerted on the phakic intraocular lens. Preferentially, the second diameter is comprised between 11.2 and 11.3 mm, more preferably, it is equivalent to 11.25 mm and it corresponds to a smaller sized ciliary sulcus, so that the rigid dome-shaped optical assembly has a size compatible with a broad range of patient eye anatomies. In particular, the rigid dome-shaped optical assembly is small enough not to itself undergo compression in an eye. The difference between the first and the second diameter can be attributed to the elongated flexible haptics, and preferentially constitutes a flexible contribution that can be contracted to fill the gap between the second diameter of the rigid dome and the size of the patient's ciliary sulcus. So, the phakic intraocular lens according to the invention is particularly well adapted to the anatomy of any eye with a planned resultant vault, ie. the above mentioned distance, not being reliant on a compression of the flexible haptics of the phakic intraocular lens.

Preferentially, a length of each of the flexible haptics, measured along a curve extending from its proximal extremity to its distal extremity, is comprised between 4 and 5 mm, more preferably between 4.4 and 4.5 mm. The elongated flexible haptics extend radially but also circumferentially beyond the optical assembly.

According to a preferred embodiment of the invention, a (whole) posterior surface of the dome is smooth and (posteriorly) concave. According to a preferred embodiment of the invention, a radius of curvature of a posterior surface of the dome is comprised between 8 and 10 mm, preferably it is equivalent to 9 mm.

Advantageously, the radius of curvature selected in this way allows the central optical part and the peripheral haptic part:
- to form a (concave) dome, the posterior surface of which is more curved than the anterior surface of a lens, and/or such that a diameter of an anterior surface of the lens is smaller than this radius of curvature;
- and consequently, to be substantially anteriorly above this anterior surface.

It is pointed out that said curvature and radius of curvature are well defined as the posterior surface of the dome is necessarily smooth and then at least regular (and/or differentiable) in a mathematical analytic sense. In particular, it has to be noticed that the posterior surface of dome at the junction between the peripheral haptic part and the central optical part does not comprise any irregularity or angular point. Preferentially, this radius of curvature is smaller than the radius of curvature of an anterior surface of the natural crystalline lens of an eye. More preferably, this radius of curvature consists in the smallest average radius of curvature of an anterior surface of a natural crystalline lens of an eye. The radii of curvature of the anterior and posterior surfaces of the phakic intraocular lens are also optimized regarding the targeted dioptric power, and in a such way that the central thickness of the central optical part is kept substantially constant across a whole diopter range. Moreover, the clear optic of the phakic intraocular lens is well defined.

Preferentially, the central optical surface is substantially convex in an anterior way and/or substantially planar and/or substantially perpendicular to the optical axis. This allows to deliver a vault without the need for compression and therefore flexing of the phakic intraocular lens anteriorly.

According to a preferred embodiment of the invention, each of the flexible haptics comprises an intermediary section comprised between its proximal extremity and its distal extremity, and apt to curve when axial and/or radial compression is exerted on each of the flexible haptics (in particular, when the phakic intraocular lens is in normal use, namely when it is in an implantation position in an eye).

This intermediary section is preferably located at a substantially equal distance from the distal and proximal extremities. Preferentially, it comprises material folds arranged to facilitate a fold in material of each of the flexible haptics, so that it curves more or less sharply when axial and/or radial pressure is exerted.

Advantageously, the flexible haptics according to this embodiment of the invention are more resistant and all the more apt to adapt to an anatomy of a ciliary sulcus of an eye. Advantageously, the curvature of the flexible haptics in their intermediary sections is used to increase the angle of contact with a ciliary sulcus, in particular, when the size of the latter is small.

Moreover, and preferentially according to this preferred embodiment, a proximal section of each of the flexible haptics, extending between its proximal extremity and its intermediary section, extends posteriorly relative to the central optical part.

Moreover, and preferentially according to this preferred embodiment, a distal section of each of the flexible haptics, extending between its intermediary section and its distal extremity, forms an angle that is preferably comprised between -15° and 15° with a perpendicular line to the optical axis.

In particular, the proximal section extends posteriorly substantially in parallel to one of the support elements whereas the distal section is not necessarily parallel to the proximal section, and this given the intermediary section in which each of the flexible haptics is apt to curve, so as to change the orientation of the proximal section with respect to the distal section. The angle formed between the distal section and a perpendicular line to the optical axis is arbitrary, although preferably comprised between -15° and 15°, in particular, in implantation position in an eye, so as to allow an orientation of the distal section that is adequate to hook into a ciliary sulcus.

Preferentially, the proximal and distal sections form another angle less than 5° in absolute value when the phakic intraocular lens is in production and has not yet been implanted in an eye, so that the insertion of the flexible haptics under the iris of a patient proves to be advantageously facilitated.

In the framework of this document, the use of the verbs "comprise", "include", "involve" or any other variant, as well as their conjugational forms, cannot in any way exclude the presence of elements other than those mentioned.

According to a preferred embodiment of the invention, each of the flexible haptics is:
- of a thickness, measured along the optical axis, that decreases from its proximal extremity to its distal extremity; and/or
- of a width, measured perpendicularly to the optical axis:
   - that decreases from its proximal extremity to its intermediary section;
   - larger at its distal extremity than at its proximal extremity.

Advantageously, the flexible haptics are thicker and wider where they are mounted on the proximal portion of the peripheral haptic part so as to make them more resistant and more solidly fixed on this proximal portion. The thickness of the flexible haptics decreases advantageously so as to allow easier insertion of their distal extremities in a ciliary sulcus.

The width is greater at their distal extremities than at their proximal extremities so that their distal extremities preferably have a foot shape, proposing a larger lateral contour in a plane perpendicular to the optical axis, and therefore a larger contact surface in a ciliary sulcus.

The flexible haptics of the phakic intraocular lens according to this embodiment are therefore not only more resistant but also offer better hooking into a ciliary sulcus and greater stability both radially and in rotation.

According to a preferred embodiment of the invention, the distal extremity of each of the flexible haptics comprises smooth (lateral and/or radial) ripples arranged to hook into a ciliary sulcus when the phakic intraocular lens is in normal use, namely when it is in an implantation position in an eye.

Advantageously, the ripples facilitate the distal extremity of each of the flexible haptics hooking into a ciliary sulcus. The ripples preferentially give a role of a pin to this distal extremity to hook more easily into a ciliary sulcus. Preferentially, these ripples are polished so that their contours are smooth and cannot irritate the ciliary sulcus.

According to a preferred embodiment of the invention, each of the flexible haptics comprises a lateral recess in its proximal extremity, which is apt to allow the latter to operate as a hinge between each of the flexible haptics and the proximal portion of the peripheral haptic part.

Advantageously, the proximal extremity is in this way apt to curve when axial and/or radial compression is exerted on the phakic intraocular lens. In this way, each of the flexible haptics is all the more flexible, solid and smooth at its proximal extremity.

Advantageously, the lateral recess plays a role of a fail safe mechanism to prevent excessive force being transmitted from the flexible haptics to the central optical part. It controls a force applied by the flexible haptics into an angle, preferably the above mentioned angle, so as to provide a adapted fixation into a ciliary sulcus and to prevent an erosion of delicate intraocular tissues.

Preferentially, the plurality of support elements comprises four support elements, and/or the plurality of flexible haptics comprises four flexible haptics, and/or the number of support elements corresponds to the number of flexible haptics.

According to an embodiment of the invention that is both specific and preferred, the phakic intraocular lens comprises:
- a first, a second, a third and a fourth support elements, inscribed within a cylinder elongated along the optical axis, centered in the central optical part, and with a diameter equal to the second diameter; the first and second support elements extending in a diametrically opposed way on either side of the optical axis; the third and fourth support elements extending in a diametrically opposed way on either side of the optical axis;
- a first, a second, a third and a fourth flexible haptics, inscribed within another cylinder elongated along the optical axis, centered in the central optical part, and with a diameter equal to the first diameter; the first and second flexible haptics extending in a diametrically opposed way on either side of the optical axis; the third and fourth flexible haptics extending in a diametrically opposed way on either side of the optical axis.

Advantageously, this arrangement of support elements and flexible haptics provides the phakic intraocular lens according to the invention with a greater stability both axially and radially as well as in rotation. In particular, the vault as predetermined space between the phakic intraocular lens and the natural crystalline lens is not dependent upon a lateral force exerted through the whole phakic intraocular lens body exerting compression. If such a lateral force occurs this phakic intraocular lens design absorbs it within the flexible haptics such that the rigid optical assembly remains unaffected by any such compression.

The extension of a pair of support elements and/or flexible haptics on either side of the optical axis, in a diametrically opposed way, can be assimilated to a collection of planar central symmetries of points of this pair of support elements and/or flexible haptics. In particular, the extension is not necessarily realized along a line or a diameter, but along a smooth curve extending symmetrically on both sides of the optical axis.

Preferentially, according to said embodiment of the invention, which is both specific and preferred, the first, second, third and fourth support elements are radially oriented to the exterior relative to the central optical part, symmetrically according to the diagonals of a first planar rectangle intersecting on the optical axis.

Preferentially, according to said embodiment of the invention, which is both specific and preferred, the distal extremities of the first, second, third and fourth flexible haptics are substantially disposed symmetrically at positions defined by vertices of a second planar rectangle of sides parallel to the sides of the first planar rectangle.

The vertices of the first plane rectangle are preferentially arranged on the cylinder. The vertices of the second plane rectangle are preferentially arranged on the other cylinder.

The ratios of the largest side of the first planar rectangle with the smallest side of the first planar rectangle, and/or of the largest side of the second planar rectangle with the smallest side of the second planar rectangle are preferably comprised between 1 and 1.5.

Preferentially, according to said embodiment of the invention, which is both specific and preferred, the proximal extremities of the first, second, third and fourth flexible haptics are substantially disposed symmetrically at positions defined by vertices of a third planar rectangle of sides parallel to the sides of the first planar rectangle.

Preferentially, according to said embodiment of the invention, which is both specific and preferred, the first, second, third and fourth flexible haptics extend respectively partially circumferentially around the first, second, third and fourth support elements.

Preferentially, according to said embodiment of the invention, which is both specific and preferred, the proximal extremities of the first and third flexible haptics, and/or the proximal extremities of the second and fourth flexible haptics, are separated by a distance less than 1 mm.

Preferentially, the length of the smallest side of the aforementioned third rectangle correspond to this distance. Preferentially, a ratio of the largest side of the third planar rectangle with the smallest side of the third planar rectangle is greater than 5, preferentially greater than 10.

Preferentially, according to said embodiment of the invention, which is both specific and preferred, the proximal extremities of the first, second, third and fourth flexible haptics are at a distance less than 2 mm from the central optical part.

Advantageously, a connection of each flexible haptic with the proximal portions of the peripheral haptic part is realized near the central optical part, so that the insertion of the flexible haptics under un iris of an eye is facilitated by means of a spatula that directs a circumferential movement from this connection to the distal extremity of each flexible haptic. Preferentially, the proximal extremities of a pair of flexible haptics are located on a same connection zone to further facilitate this insertion.

According to a preferred embodiment of the invention, the phakic intraocular lens comprises a flexible and biocompatible material comprising between 30 and 40% water, a Young module of which being less than 1 GPa.

Preferentially, this material comprises 36 to 38% water. Preferentially, this material has a Young module comprised between 0.25 and 0.75 GPa, more preferably between 0.4 and 0.6 GPa, even more preferably, it is 0.5 GPa.

This material is preferentially more flexible than a material usually used in intraocular lens design. This material is preferentially a polymacon.

Preferentially, the phakic intraocular lens according to the invention is extensively polished on each of its external surfaces, in particular, on the anterior, posterior and lateral surfaces, in order to avoid one side or a cutting edge of a flexible haptic, of the peripheral haptic part or the central optical part, to be able to be an irritant for the tissues of a posterior chamber of a patient's eye when a phakic intraocular lens is in an implantation position.

Moreover, according to a preferred embodiment of the invention, the phakic intraocular lens comprises a through bore extending between the anterior and posterior surfaces and arranged to allow a fluid flow.

Preferentially, the fluid flow is a flow of a liquid between the anterior and posterior chambers of an eye when the phakic intraocular lens is in normal use in an eye. This bore advantageously allows to prevent a second and artificial posterior chamber to be created with a resultant limitation of a fluid flow between a space anterior and a space posterior to the phakic lens regardless of the actual size of either of these spaces. It makes possible to guarantee a full and permanent fluid communication between the anterior and posterior chambers of the eye. Preferentially, the through bore is located in a center of the central optical part, more preferably at least partially at its intersection with the optical axis.

The invention advantageously allows a choice of central optical part lens that is best adapted to a defect in vision that is to be corrected in a patient.

In particular, according to an embodiment of the invention, the central optical part lens consists in a monofocal lens that allows at least one correction from among: a correction of myopia, a correction of hypermetropia, and a correction of corneal astigmatism. According to a particular embodiment of the invention, the lens consists in a refractive or diffractive lens at extended depth of focus. Preferentially, the lens is selected according to the state of the art.

In the framework of the present document, an "optical axis" of an eye consists preferentially in a vector crossing the eye from one side to the other, directed by its anterior segment, comprising successively, the cornea, the iris and the lens, to its posterior segment, comprising especially the retina. For a phakic intraocular lens according to the invention in an implantation position in an eye, the optical axis of the eye is directed from the anterior surface to the posterior surface and preferentially corresponds to the optical axis defined intrinsically with respect to the phakic intraocular lens. In particular, the term optical axis is presently and preferentially used in this document as the reference axis with respect to the eye and/or to the phakic intraocular lens.

In the framework of the present document, an "anterior" (or respectively, "posterior") side and/or surface of part of an eye or an intraocular lens consists preferentially in a side and/or surface located upstream (or respectively, downstream) of the part of the eye or the intraocular lens relative to the vector defined by the optical axis. By way of example, in an eye, the iris is located anteriorly with respect to the lens; a posterior surface of the iris is therefore a part of the iris that is the closest to the lens. Likewise, when a first part of an eye or of an intraocular lens is anteriorly (or respectively, posteriorly) above a second part of an eye or an intraocular lens, it follows that this first part is located anteriorly (or respectively, posteriorly) with respect to this second part.

The aforementioned notions of anteriority, posteriority or even of an optical axis relative to parts of an eye and/or an intraocular lens are well known to person skilled in the art.

In particular, the phakic intraocular lens according to the present invention is configured to be positioned in the posterior chamber of an eye, so that its anterior surface is at least partially facing the iris of the eye and so that its posterior surface is at least partially facing the lens of the eye.

In the framework of the present document, it is said that part of a phakic intraocular lens extends "radially outward" when it extends preferentially according to vectors perpendicular to the optical axis, directed from a point in common with the optical axis to points of a circle centered at this common point. Likewise, it is said that a part of a phakic intraocular lens extends "circumferentially" when it extends preferentially along at least an arc of a circle on a plane perpendicular to the optical axis and centered on an intersection point of the plane and the optical axis. These notions of radial and circumferential extensions refer to a system of polar coordinates in each plane perpendicular to the optical axis.

It is well known by a person skilled in the art that the adjective "distal" refers to a part of a portion of a body the furthest form a reference organ or from a trunk of a body, and that the adjective "proximal" refers to a portion of a part of a body the closest to a reference organ or to a trunk of a body. In the framework of the present document, these two definitions will apply preferentially to parts of an eye and/or a phakic intraocular lens according to the invention, relative to a distance with respect to the referential optical axis. For example, and preferentially, a proximal portion of a phakic intraocular lens according to the invention comprises a central optical part and/or a part of the phakic intraocular lens around a central part, and a distal portion of a phakic intraocular lens according to the invention comprises an external lateral edge of the phakic intraocular lens apt to reach part of an eye.

In the framework of this document, the use of the indefinite article "a", "an" or the definite article "the" to introduce an element does not exclude the presence of a plurality of these elements.

### Brief description of the figures

Other characteristics and advantages of the present invention will appear on reading the detailed descript that follows, for the understanding of which, it is referred to the attached figures among which:
- figure 1A illustrates a first simplified planar representation of the top of a posterior chamber phakic intraocular lens according to the invention;
- figure 1B illustrates a first simplified planar representation provided with additional abstract geometric guiding marks;
- figure 2 illustrates a second simplified planar representation of the side of the posterior chamber phakic intraocular lens illustrated in figure 1;
- figure 3 illustrates a third simplified planar representation of the front of the posterior chamber phakic intraocular lens illustrated in figure 1;
- figure 4A illustrates a first simplified three-dimensional representation of the side and partially of the posterior surface of the posterior chamber phakic intraocular lens illustrated in figure 1;
- figure 4B illustrates a second simplified three-dimensional representation of the anterior surface of the posterior chamber phakic intraocular lens illustrated in figure 1;
- figure 5 illustrates a simplified cross-sectional view of part of an eye in which a posterior chamber phakic intraocular lens according to the invention has been fitted;
- figure 6A illustrates a simplified planar representation of the top of a posterior chamber phakic intraocular lens according to the invention;
- figure 6B illustrates a simplified planar representation of the top of a posterior chamber phakic intraocular lens according to the invention;
- figure 7 illustrates a simplified planar representation of the side of a posterior chamber phakic intraocular lens according to the invention;
- figure 8 illustrates a simplified planar representation of the top of a distal extremity of one of the flexible haptics of a posterior chamber phakic intraocular lens according to the invention;
- figure 9 illustrates a simplified planar representation of the top of a posterior chamber phakic intraocular lens according to the invention;
- figure 10 illustrates a graphic representation of an axial position of a posterior chamber phakic intraocular lens according to the invention on the basis of a ciliary sulcus diameter.

The drawings in the figures are not to scale. Generally, similar elements are denoted by similar references in the figures. In the framework of the present document, identical or analogous elements may have the same references. Moreover, the presence of reference numbers in the drawings cannot be considered to be limiting, comprising when these numbers are indicated in the claims.

### Detailed description of specific embodiments of the invention

This part presents a detailed description of preferred embodiments of the present invention. It is described with specific embodiments and references to figures, but the invention in not limited by these. In particular, the drawings or figures described below are only schematic and are not limiting in any way.

The geometry elements designated by references X, Y, Z, K, d, R, S, T, C, r1, r2, s1, s2, s3, s4, t1, t2, t3 and t4 are represented in some figures below, purely as an illustration in order to quantify and/or visualize technical properties of embodiments of the invention. In particular, these elements are abstract and do not correspond to concrete material objects.

The present invention provides a posterior chamber phakic intraocular lens 1 that is both adapted to any eye anatomy and stable in an implantation position in an eye 9, both axially along the optical axis Z, radially and in rotation in a plane perpendicular to the optical axis Z based on the vectors X and Y. The optical axis Z is directed from an anterior surface 11 of the phakic intraocular lens 1 to a posterior surface 12 of the phakic intraocular lens 1.

As illustrated in the present figures, the phakic intraocular lens 1 according to the invention comprises a central optical part 2 extending radially relative to the optical axis Z, and in a substantially planar and/or substantially convex way, with a diameter comprised between 5.5 and 6.5 mm, preferentially equivalent to 6 mm. It comprises an through bore 21 elongated along the optical axis Z and extending between anterior 11 and posterior 12 surfaces so as to allow fluid communication between these surfaces.

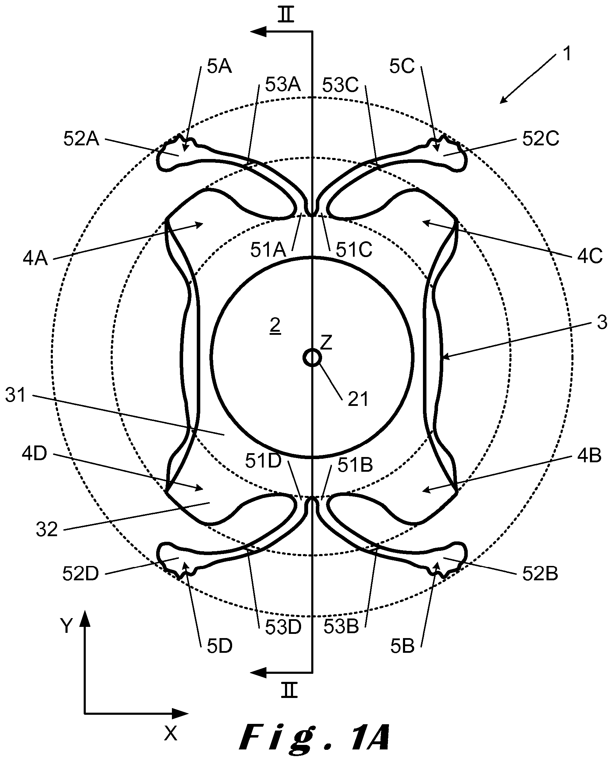

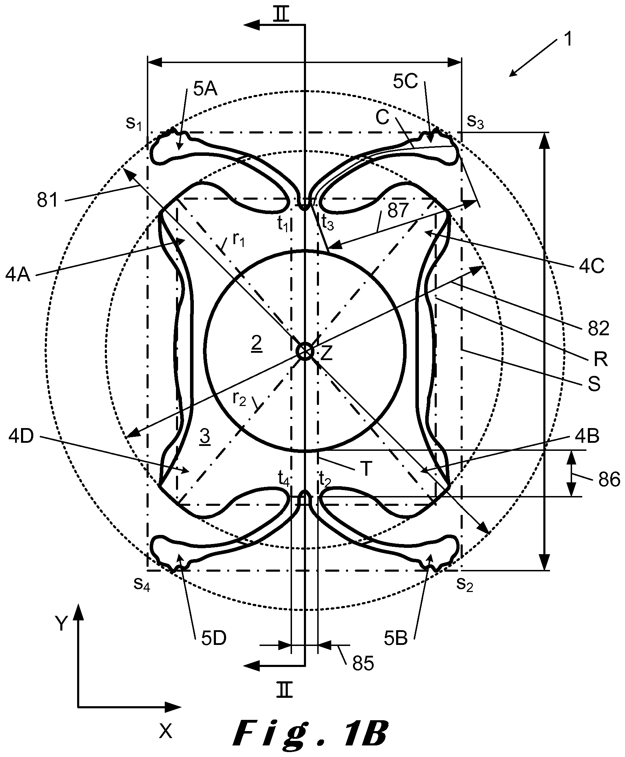

As illustrated in figures 1A and 1B, the phakic intraocular lens 1 comprises a peripheral haptic part 3 extending radially outward relative to said central optical part 2, and comprising;
- a proximal portion 31 extending circumferentially and symmetrically around and from said central optical part 2;
- a distal portion 32 prolonging at least partially in a radial way, the proximal portion 31 and comprising a plurality of support elements 4A-D extending radially outward along diagonals r1 and r2 of a first planar rectangle R perpendicular to the optical axis Z,
and also extending posteriorly relative to the central optical part 2; so that the central optical parts 2 and peripheral haptic parts 3 form a dome K supported by broad feet consisting in support elements 4A-D.

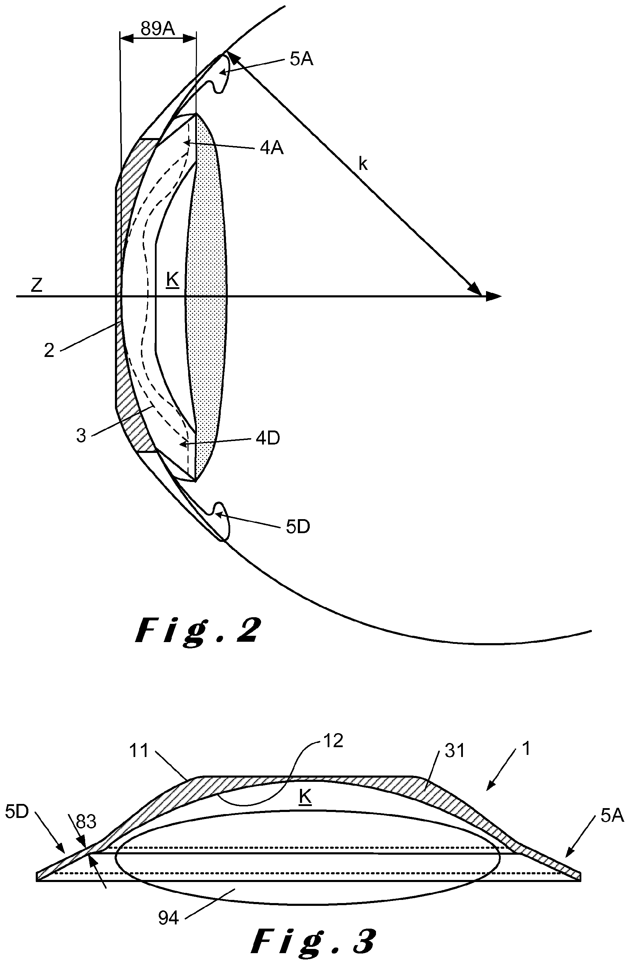

Dome K is represented in Figure 2. A sufficient wall thickness confers rigidity to the dome K so that it is resistant under axial and/or radial compression. A posterior surface of the dome K is curved and its preferential radius of curvature k is approximately 9 mm, so that the dome K can be inscribed into a sphere with radius k as illustrated in figure 2. A height 89A of the dome K, measured along the optical axis Z, and as illustrated in figure 2, and said "inherent vault", constitute a height of a vault inherent to the phakic intraocular lens 1. It accepts a preferential value comprised between 1.5 and 2.2 mm, more preferentially between 1.7 and 2 mm, even more preferentially it is 1.87 mm. The width of a base of dome K corresponds to a second diameter 82 of the central optical 2 and peripheral haptic 3 parts. This second diameter 82 is approximately 11.25 mm.

As presented in detail in the summary of the invention, these values are selected so that the assembly composed of the central optical 2 and peripheral haptic 3 parts are apt to constitute a sufficiently rigid and sufficiently broad structure to surround and sit anteriorly above a lens 94 of an eye, as diagramed in figure 3, and thereby to be implanted in a very broad range of anatomies of eye posterior chambers, while being stable in parallel to the optical axis Z.

As illustrated in figures 1A and 1B, the phakic intraocular lens 1 also comprises a plurality of flexible haptics 5A-D, each being elongated in form along a curve that extends both radially outward with respect to the central optical part 2, and circumferentially around one of the support elements 4A-D. For example, as illustrated in figure 1B, the flexible haptic 5C is elongated and follows the curve C, and extends circumferentially around the support element 4C. In this way, the flexible haptics 5A-D extend radially beyond the central optical 2 and peripheral haptic 3 so as to inscribe in a cylinder of a first diameter 81 with a value preferentially comprised between 14.5 and 15 mm, more preferentially 14.7 mm. Each of the flexible haptics 5A-D has a length 87 comprised between 4.2 and 4.7 mm, more preferentially 4.45 mm.

Each of the flexible haptics 5A-D comprises a proximal extremity 51A-D mounted on the proximal portion 31, disposed at a vertex t1-4 of a planar rectangle T perpendicular to the optical axis Z, the smallest side of which, separating the vertices (t2, t4) and (t1, t3), has a length 85 comprised between 0.5 mm and 1 mm, more preferentially between 0.6 and 0.8 mm, and the largest side, separating the vertices (t1, t4) and (t3, t2), has a length comprised between 7.5 and 8.5 mm, more preferentially between 7.9 and 8.3 mm. Each of the flexible haptics 5A-D comprises a distal extremity 52A-D in foot form, disposed at a vertex s1-4 of a planar rectangle S perpendicular to the optical axis Z, the smallest side of which, separating the vertices (s2, s4) and (s1, s3), has a length comprised between 8.5 mm and 9 mm, more preferentially equivalent to 8.8 mm, and the largest side, separating the vertices (s1, s4) and (s3, s2), has a length comprised between 12 and 13 mm, more preferentially between 12.3 and 12.6 mm, more preferentially equivalent to 12.43 mm, these data being considered for flexible haptics 5A-D in production, not implanted, without axial or radial compression exerted on the phakic intraocular lens 1.

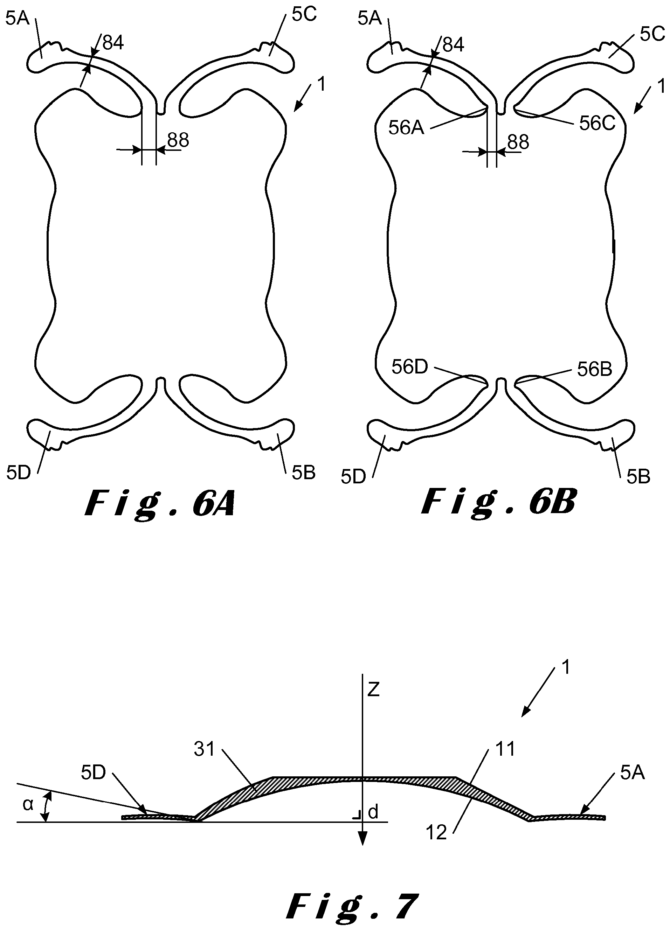

Each of the flexible haptics 5A-D comprises an intermediary section 53A-D between the proximal extremity 51A-D and the distal extremity 52A-D, preferentially in its middle, comprising material folds configured to allow a fold and/or a curve of each of the flexible haptics 5A-D in this intermediary section when compression is exerted axially and/or radially on the phakic intraocular lens 1, so that an angle α between a line d perpendicular to the optical axis Z and a direction of extensions between the distal extremities 52A-D and the intermediary sections 53A-D is preferentially apt to be comprised between -15° and 15°, as illustrated in figure 7.

The choice of the aforementioned length 85 and a distance 86 between the proximal extremities 51A-D and the central optical part 2 preferentially comprised between 1 and 1.5 mm is used to guide the flexible haptics 5A-D advantageously when the phakic intraocular lens 1 is implanted under an iris, from a same limited zone of the peripheral haptic part 3 comprising the connections with two of the proximal extremities 51A-D.

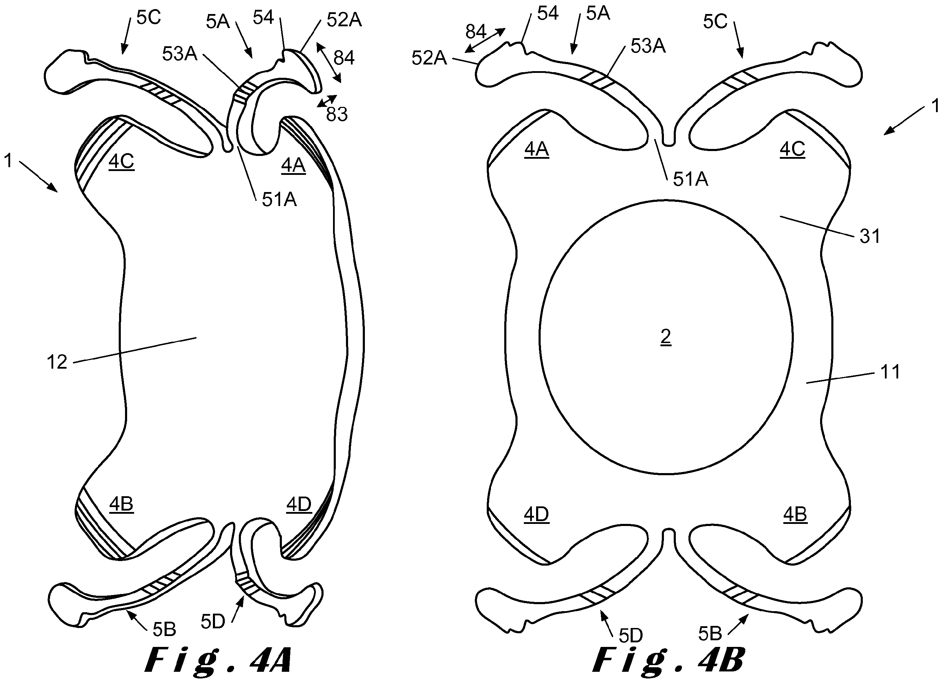

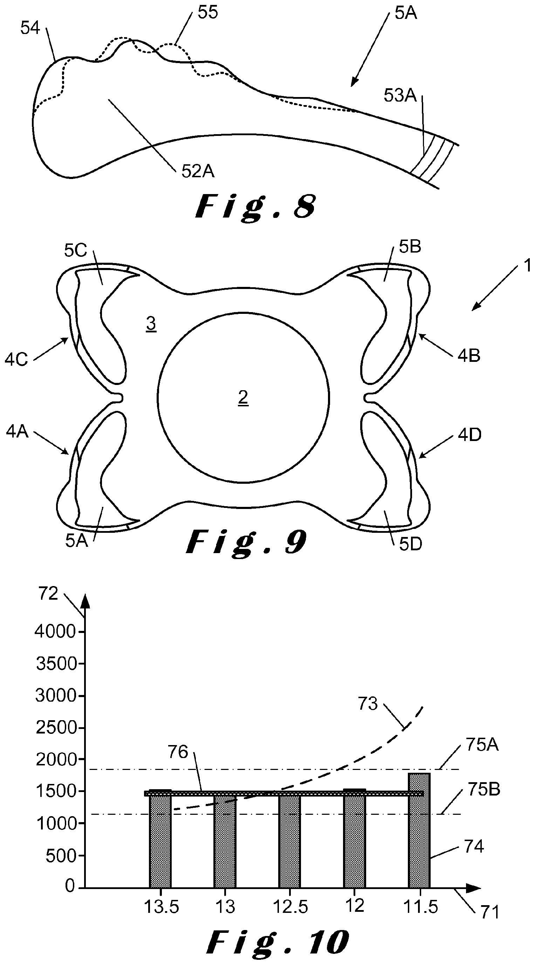

These haptics 5A-D are constituted from a flexible and resistant material comprising preferably 38% water, which combined with their geometry gives them their flexibility. As represented in figures 3, 4A-B and 6A-B, the flexible haptics 5A-D have a thickness 83 that decreases from the proximal extremities 51A-D to the distal extremities 52A-D and a width 84 that is greater, preferentially twice as much, at the distal extremities 52A-D than on the proximal sections comprised respectively between the proximal extremities 51A-D and the intermediary sections 53A-D, so that the distal extremities 52A-D have a form of flat feet adapted to be inserted into and to hook into a ciliary sulcus of an eye. This form and this technical effect are preferentially accentuated by smooth ripples 54, 55 on a lateral surface of the distal extremities 52A-D. Two examples of such ripples 54 and 55 are represented in figure 8 on the distal extremity 52A, and without limitation as to the choice of the distal extremity. These ripples 54 are spaced at a greater distance from each other and are less curved that the ripples 55.

The geometry of the proximal extremities 51A-D confers great solidity and resistance to the connections between them and the proximal portion 31 of the peripheral haptic part 3. These connections have a width 88 illustrated in figures 6A-B, and preferentially comprised between 0.8 and 0.4 mm. As represented more specifically in figure 6B, they are likely to be adjacent to lateral recesses 56A-D of the proximal extremities 51A-S, so that the width 88 is then preferentially comprised between 0.4 and 0.5 mm. Without these lateral recesses, as illustrated specifically in figure 6A, width 88 is preferentially comprised between 0.7 and 0.8 mm. These recesses 56A-D allow the proximal extremities 51A-D to increase their flexibility so that the function as hinges between the flexible haptics 5A-D and the proximal portion 31.

According to a particular embodiment of the invention illustrated in figure 9, the flexible haptics 5A-D are apt to be less partially folded on the support element 4A-D. This closed configuration is likely to be advantageous prior to an injection of the phakic intraocular lens 1 into a posterior chamber of an eye. Generally, it seems obvious for the person skilled in the art that the flexibility of the flexible haptics 5A-D is used to arrange them in different ways before, during and after the phakic intraocular lens 1 is implanted.

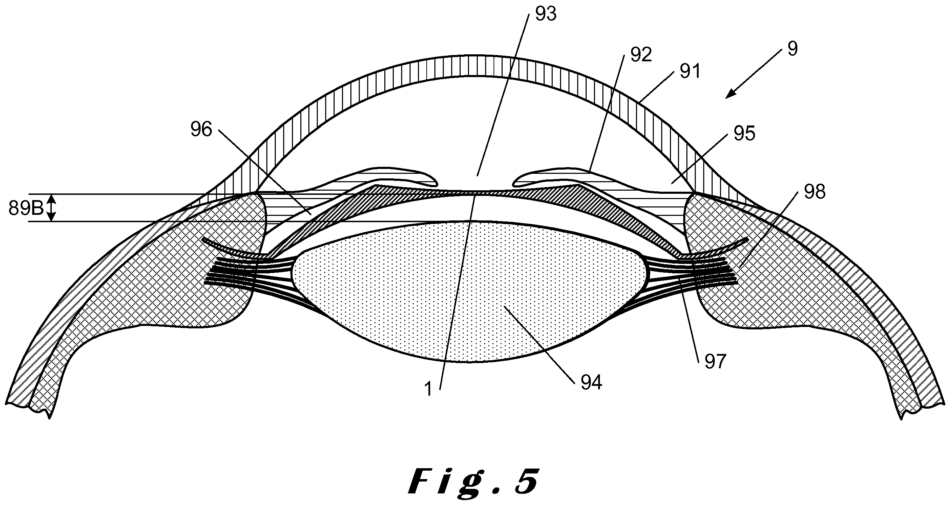

Figure 5 represents a cross-section of an eye 9, in which a phakic intraocular lens 1 is implanted according to the invention. On this cross-section the following are represented: a cornea 91, an iris 92, a pupil 93, a lens 94, and anterior chamber 95, a posterior chamber 96, a ciliary zonule 97 and a ciliary sulcus 98 of the eye 9. The phakic intraocular lens 1 is placed in the posterior chamber 96. The support elements 4A-D of the peripheral haptic part 3 rest on the ciliary zonule 97 while the flexible haptics 5A-D are made to hook into the ciliary sulcus 98 as described and commented on in the summary of the invention. A distance of security 89B, referred to as the vault, between the lens 94 and the posterior surface of the phakic intraocular lens 1 measured along the optical axis Z is preferentially comprised and adjustable between 350 and 700 microns. The double structure of haptics 4A-D and 5A-D allow axial and radial stabilisation and stabilisation in rotation of the phakic intraocular lens 1 in its implantation position.

Figure 10 illustrates a graphic representation 74 of an axial position of a posterior chamber phakic intraocular lens according to the invention 1, indicated on the axis 72 and measured in microns on the basis of a ciliary sulcus diameter, indicated on axis 71 and measured in millimeters. A desirable equilibrium position is represented by a line 76 at 1500 microns measured on the axis 72, this value corresponding to an aforementioned distance 89B of 500 microns. An axially accepted margin of variation according to the optical axis Z around this equilibrium position 76 is of 350 microns. This margin is represented at its high and low limits by two dotted lines 75A and 75B respectively. The graph 74 shows that the axial displacements of the phakic intraocular lens 1 on the basis of the size of the ciliary sulcus vary very little and remain within the accepted values between lines 75A-B for a broad range of sizes of ciliary sulci comprised between 11.5 and 13.5 mm. The curve 73 illustrated by a dotted line represents a measurement trend of an average axial displacement of an average posterior chamber phakic intraocular lens according to the prior art and with a diameter of 12.6 mm. Comparison of chart 74 and the tendency curve 73 illustrates the performances and the improvements in terms of axial stability of the phakic intraocular lens 1 according to the invention.

In other words, the present invention relates to a posterior chamber phakic intraocular lens 1 comprising a central optical part 2, a peripheral haptic part 3 comprising a plurality of support elements 4A-D arranged to lie on a ciliary zonule of one eye, and a plurality of flexible elongated haptics 5A-D arranged to hook into a ciliary sulcus of the eye.

The present invention was described in relation to the specific embodiments which have a value that is purely illustrative and should not be considered to be limiting. Generally speaking, it seems obvious for the person skilled in the art that the present invention is not limited to the examples illustrated and/or described above. The invention comprises each of the new characteristics described throughout the present document, as well as all their combinations.

## Claims

1. Posterior chamber phakic intraocular lens (1) comprising:
- an anterior surface (11) and a posterior surface (12);
- a central optical part (2) comprising a lens, and extending radially relative to an optical axis (Z) directed from said anterior surface (11) to said posterior surface (12);
- a peripheral haptic part (3) extending radially outward relative to said central optical part (2);
- a plurality of elongated flexible haptics (5A-D); **characterized in that**:
- said peripheral haptic part (3) comprises:
• a proximal portion (31) mounted on said central optical part (2) and extending circumferentially and symmetrically around said central optical part (2);
• a distal portion (32) mounted on said proximal portion (31) and comprising a plurality of support elements (4A-D) extending both radially outward and posteriorly relative to said central optical part (2); said support elements (4A-D) being configured for supporting said phakic intraocular lens (1) on a ciliary zonule (97) when it is in normal use in an eye (9);
said central optical part (2) and said peripheral haptic part (3) forming a dome (K);
- each of said flexible haptics (5A-D) extends both partially circumferentially around one of said support elements (4A-D), and radially outward relative to said central optical part (2);
a first diameter (81) of said phakic intraocular lens (1) being strictly greater than a second diameter (82) of an optical assembly consisting in said central optical part (2) and said peripheral haptic part (3); each of said flexible haptics (5A-D) extending at least partially between said first (81) and second (82) diameters;
each of said flexible haptics (5A-D) comprising:
• a proximal extremity (51A-D) mounted on said proximal portion (31) of said peripheral haptic part (3);
• a free distal extremity (52A-D) for hooking said phakic intraocular lens (1) into a ciliary sulcus (98) when it is in normal use in an eye (9).

2. Phakic intraocular lens (1) according to the preceding claim, **characterized in that** each of said flexible haptics (5A-D) comprises an intermediary section (53A-D) comprised between its proximal extremity (51A-D) and its distal extremity (52A-D), and apt to curve when axial and/or radial compression is exerted on each of said flexible haptics (5A-D);
and **in that**:
- a proximal section of each of said flexible haptics (5A-D), extending between its proximal extremity (51A-D) and its intermediary section (53A-D), extends posteriorly relative to said central optical part (2);
- a distal section of each of said flexible haptics (5A-D), extending between its intermediary section (53A-D) and its distal extremity (52A-D), forms an angle (α) comprised between -15° and 15° with a perpendicular line (d) to said optical axis (Z).

3. Phakic intraocular lens (1) according to the preceding claim, **characterized in that** each of said flexible haptics (5A-D) is:
- of a thickness (83), measured along said optical axis (Z), that decreases from its proximal extremity (51A-D) to its distal extremity (52A-D);
- of a width (84) measured perpendicularly to said optical axis (Z):
• that decreases from its proximal extremity (51A-D) to its intermediary section (53A-D);
• larger at its distal extremity (52A-D) than at its proximal extremity (51A-D).

4. Phakic intraocular lens (1) according to any one of the preceding claims, **characterized in that** said free distal extremity (52A-D) comprises smooth ripples (54, 55) arranged to hook into a ciliary sulcus (98) when said phakic intraocular lens (1) is in normal use in an eye (9).

5. Phakic intraocular lens (1) according to any one of the preceding claims, **characterized in that** each of said flexible haptics (5A-D) comprises a lateral recess (56A-D) in its proximal extremity (51A-D) apt to allow the latter to operate as a hinge between each of said flexible haptics (5A-D) and said proximal portion (31) of said peripheral haptic part (3).

6. Phakic intraocular lens (1) according to any one of the preceding claims, **characterized in that** it comprises:
- a first (4A), a second (4B), a third (4C) and a fourth (4D) support elements inscribed within a cylinder elongated along said optical axis (Z), centered in said central optical part (2), and with a diameter equal to said second diameter (82);
said first (4A) and second (4B) support elements extending in a diametrically opposed way on either side of said optical axis (Z);
said third (4C) and fourth (4D) support elements extending in a diametrically opposed way on either side of said optical axis (Z);
- a first (5A), a second (5B), a third (5C) and a fourth (5D) flexible haptics inscribed within another cylinder elongated along said optical axis (Z), centered in said central optical part (2), and with a diameter equal to said first diameter (81);
said first (5A) and second (5B) flexible haptics extending in a diametrically opposed way on either side of said optical axis (Z); said third (5C) and fourth (5D) flexible haptics extending in a diametrically opposed way on either side of said optical axis (Z).

7. Phakic intraocular lens (1) according to the preceding claim, **characterized in that**:
- said first (4A), second (4B), third (4C) and fourth (4D) support elements are radially oriented to the exterior relative to said central optical part (2), symmetrically according to the diagonals (r1, r2) of a first planar rectangle (R) intersecting on said optical axis (Z);
- the distal extremities (52A-D) of said first (5A), second (5B), third (5C) and fourth (5D) flexible haptics are substantially disposed symmetrically at positions defined by vertices (s1, s2, s3, s4) of a second planar rectangle (S) of sides parallel to the sides of said first planar rectangle (R);
- the proximal extremities (51A-D) of said first (5A), second (5B), third (5C) and fourth (5D) flexible haptics are substantially disposed symmetrically at positions defined by vertices (t1, t2, t3, t4) of a third planar rectangle (T) of sides parallel to the sides of said first planar rectangle (R);
- said first (5A), second (5B), third (5C) and fourth (5D) flexible haptics extend partially circumferentially around said first (4A), second (4B), third (4C) and fourth (4D) support elements respectively.

8. Phakic intraocular lens (1) according to any one of the two preceding claims, **characterized in that**:
- the proximal extremities (51A, 51C) of said first (5A) and third (5C) flexible haptics are separated by a distance (85) less than 1 mm;
- the proximal extremities (51B, 51D) of said second (5B) and fourth (5D) flexible haptics are separated by a distance (85) less than 1 mm;
- the proximal extremities (51A-D) of said first (5A), second (5C), third (5C) and fourth (5D) flexible haptics are at a distance (86) less than 2 mm from the central optical part (2).

9. Phakic intraocular lens (1) according to any one of the preceding claims, **characterized in that**:
- said first diameter (81) is comprised between 14 and 15 mm;
- said second diameter (82) is comprised between 11 and 12 mm.

10. Phakic intraocular lens (1) according to any one of the preceding claims, **characterized in that** a length (87) of each of said flexible haptics, measured along a curve (C) extending from its proximal extremity (51A-D) to its distal extremity (52A-D), is comprised between 4 and 5 mm.

11. Phakic intraocular lens (1) according to any one of the preceding claims, **characterized in that** a radius of curvature (k) of a posterior surface of said dome (K) is comprised between 8 and 10 mm.

12. Phakic intraocular lens (1) according to any one of the preceding claims, **characterized in that** a posterior surface of said dome (K) is smooth and concave.

13. Phakic intraocular lens (1) according to any one of the preceding claims, **characterized in that** it comprises a flexible and biocompatible material comprising between 30 and 40% water, a Young module of which being less than 1 GPa.

14. Phakic intraocular lens (1) according to any one of the preceding claims, **characterized in that** the central optical part (2) comprises a through bore (21) extending between said anterior (11) and posterior (12) surfaces and arranged to allow a fluid flow.

15. Phakic intraocular lens (1) according to any one of the preceding claims, **characterized in that** said lens consists in a refractive or diffractive lens at extended depth of focus.

## Patentansprüche

1. Phakische Hinterkammer-Intraokularlinse (1), die umfasst:
- eine vordere Oberfläche (11) und eine hintere Oberfläche (12);
- ein zentrales optisches Teil (2), das eine Linse umfasst und sich radial in Bezug zu einer optischen Achse (Z), die von der vorderen Oberfläche (11) zu der hinteren Oberfläche (12) gerichtet ist, erstreckt;
- ein peripheres haptisches Teil (3), das sich radial in Bezug zu der zentralen optischen Achse (2) nach außen erstreckt;
- eine Vielzahl länglicher biegsamer Haptiken (5A-D);
**dadurch gekennzeichnet, dass**:
- das periphere haptische Teil (3) umfasst:
• einen proximalen Abschnitt (31), der auf das zentrale optische Teil (2) montiert ist und sich umfänglich und symmetrisch um das zentrale optische Teil (2) erstreckt;
• einen distalen Abschnitt (32), der auf den proximalen Abschnitt (31) montiert ist und eine Vielzahl von Tragelementen (4A-D) umfasst, die sich sowohl radial auswärts als auch rückwärts in Bezug zu dem zentralen optischen Teil (2) erstrecken;
wobei die Tragelemente (4A-D) zum Tragen der phakischen Intraokularlinse (1) auf einer ziliaren Zonula (97), wenn sie in normaler Nutzung in einem Auge (9) ist, konfiguriert sind;
wobei das optische Teil (2) und das periphere haptische Teil (3) eine Kuppel (K) bilden;
- wobei sich jede der biegsamen Haptiken (5A-D) sowohl teilweise umfänglich um eines der Tragelemente (4A-D) als auch radial auswärts in Bezug zu dem zentralen optischen Teil (2) erstreckt;
wobei ein erster Durchmesser (81) der phakischen Intraokularlinse (1) strikt größer ist als ein zweiter Durchmesser (82) einer optischen Anordnung, die aus dem zentralen optischen Teil (2) und dem peripheren haptischen Teil (3) besteht; wobei sich jede der biegsamen Haptiken (5A-D) mindestens teilweise zwischen dem ersten (81) und dem zweiten (82) Durchmesser erstreckt;
wobei jede der biegsamen Haptiken (5A-D) umfasst:
• ein proximales Ende (51A-D), das auf den proximalen Abschnitt (31) des peripheren haptischen Teils (3) montiert ist;
• ein freies distales Ende (52A-D) zum Einhaken der phakischen Intraokularlinse (1) in einen ziliaren Sulkus (98), wenn sie in normaler Verwendung in einem Auge (9) ist.

2. Phakische Intraokularlinse (1) nach dem vorstehenden Anspruch, **dadurch gekennzeichnet, dass** jede der biegsamen Haptiken (5A-D) einen Zwischenteilabschnitt (53A-D) umfasst, der zwischen ihrem proximalen Ende (51A-D) und ihrem distalen Ende (52A-D) liegt und geeignet ist, sich zu krümmen, wenn axiale und/oder radiale Kompression auf jede der biegsamen Haptiken (5A-D) ausgeübt wird.
und dass:
- sich ein proximaler Teilabschnitt jeder der biegsamen Haptiken (5A-D), der sich zwischen ihrem proximalen Ende (51A-D) und ihrem Zwischenteilabschnitt (53A-D) erstreckt, sich rückwärts in Bezug zu dem zentralen optischen Teil (2) erstreckt;
- sich ein distaler Teilabschnitt jeder der biegsamen Haptiken (5A-D), der sich zwischen ihrem Zwischenteilabschnitt (53A-D) und ihrem distalen Ende (52A-D) erstreckt, einen Winkel (α) bildet, der zwischen -15° und 15° mit einer senkrechten Linie (d) zu der optischen Achse (Z) erstreckt.

3. Phakische Intraokularlinse (1) nach dem vorstehenden Anspruch, **dadurch gekennzeichnet, dass** jede der biegsamen Haptiken (5A-D):
- eine Dicke (83) entlang der optischen Achse (Z) gemessen aufweist, die von ihrem proximalen Ende (51A-D) zu ihrem distalen Ende (52A-D) abnimmt;
- eine Breite (84) senkrecht zu der optischen Achse (Z) gemessen aufweist:
• die von ihrem proximalen Ende (51A-D) zu ihrem Zwischenteilabschnitt (53A-D) abnimmt;
• an ihrem distalen Ende (52A-D) größer ist als an ihrem proximalen Ende (51A-D).

4. Phakische Intraokularlinse (1) nach einem der vorstehenden Ansprüche, **dadurch gekennzeichnet, dass** das freie distale Ende (52A-D) glatte Kräuselungen (54, 55) umfasst, die eingerichtet sind, um in einen ziliaren Sulkus (98) einzuhaken, wenn die phakische Intraokularlinse (1) in normaler Verwendung in einem Auge (9) ist.

5. Phakische Intraokularlinse (1) nach einem der vorstehenden Ansprüche, **dadurch gekennzeichnet, dass** jede der biegsamen Haptiken (5A-D) eine seitliche Vertiefung (56A-D) in ihrem proximalen Ende (51A-D) umfasst, die dazu geeignet ist, es der Letzteren zu erlauben, als ein Scharnier zwischen jeder der biegsamen Haptiken (5A-D) und dem proximalen Abschnitt (31) des peripheren haptischen Teils (3) zu arbeiten.

6. Phakische Intraokularlinse (1) nach einem der vorstehenden Ansprüche, **dadurch gekennzeichnet, dass** sie umfasst:
- ein erstes (4A), ein zweites (4B), ein drittes (4C) und ein viertes (4D) Tragelement, die in einem Zylinder umschrieben sind, der sich entlang der optischen Achse (Z), in dem zentralen optischen Teil (2) zentriert ist und mit einem Durchmesser gleich dem zweiten Durchmesser (82) ausdehnt;
wobei sich das erste (4A) und das zweite (4B) Tragelement in einer diametral entgegengesetzten Art auf beiden Seiten der optischen Achse (Z) erstrecken;
wobei sich das dritte (4C) und das vierte (4D) Tragelement in einer diametral entgegengesetzten Art auf beiden Seiten der optischen Achse (Z) erstrecken;
- eine erste (5A), eine zweite (5B), eine dritte (5C) und eine vierte (5D) biegsame Haptik, die in einem anderen Zylinder umschrieben sind, der sich entlang der optischen Achse (Z), in dem zentralen optischen Teil (2) zentriert und mit einem Durchmesser gleich dem ersten Durchmesser (81) ausdehnt;
wobei sich die erste (5A) und die zweite (5B) biegsame Haptik in einer diametral entgegengesetzten Art auf beiden Seiten der optischen Achse (Z) erstrecken;
wobei sich die dritte (5C) und die vierte (5D) biegsame Haptik in einer diametral entgegengesetzten Art auf beiden Seiten der optischen Achse (Z) erstrecken.

7. Phakische Intraokularlinse (1) nach dem vorstehenden Anspruch, **dadurch gekennzeichnet, dass**:
- das erste (4A), das zweite (4B), das dritte (4C) und das vierte (4D) Tragelement radial in Bezug zu dem zentralen optischen Teil (2) nach außen, symmetrisch gemäß den Diagonalen (r1, r2) eines ersten planaren Rechtecks (R), das die optische Achse (Z) schneidet, ausgerichtet sind;
- die distalen Enden (52A-D) der ersten (5A), zweiten (5B), dritten (5C) und vierten (5D) biegsamen Haptik im Wesentlichen symmetrisch an Positionen angeordnet sind, die von Eckpunkten (s1, s2, s3, s4) eines zweiten planaren Rechtecks (S) von Seiten parallel zu den Seiten des ersten planaren Rechtecks (R) definiert sind;
- die proximalen Enden (51A-D) der ersten (5A), zweiten (5B), dritten (5C) und vierten (5D) biegsamen Haptik im Wesentlichen symmetrisch an Positionen angeordnet sind, die von Eckpunkten (t1, t2, t3, t4) eines dritten planaren Rechtecks (T) von Seiten parallel zu den Seiten des ersten planaren Rechtecks (R) definiert sind;
- wobei sich die erste (5A), zweite (5B), dritte (5C) und vierte (5D) biegsame Haptik teilweise umfänglich jeweils um das erste (4A), zweite (4B), dritte (4C) und vierte (4D) Tragelement erstrecken.

8. Phakische Intraokularlinse (1) nach einem der zwei vorstehenden Ansprüche, **dadurch gekennzeichnet, dass**:
- die proximalen Enden (51A, 51C) der ersten (5A) und dritten (5C) biegsamen Haptik von einem Abstand (85) kleiner als 1 mm getrennt sind;
- die proximalen Enden (51B, 51D) der zweiten (5B) und vierten (5D) biegsamen Haptik von einem Abstand (85) kleiner als 1 mm getrennt sind;
- die proximalen Enden (51A-D) der ersten (5A), der zweiten (5B), der dritten (5C) und der vierten (5D) biegsamen Haptik in einem Abstand (86) kleiner als 2 mm von dem zentralen optischen Teil (2) liegen.

9. Phakische Intraokularlinse (1) nach einem der vorstehenden Ansprüche, **dadurch gekennzeichnet, dass**:
- der erste Durchmesser (81) zwischen 14 und 15 mm liegt;
- der zweite Durchmesser (82) zwischen 11 und 12 mm liegt.

10. Phakische Intraokularlinse (1) nach einem der vorstehenden Ansprüche, **dadurch gekennzeichnet, dass** eine Länge (87) jeder der biegsamen Haptiken, entlang einer Krümmung (C) gemessen, die sich von ihrem proximalen Ende (51A-D) zu ihrem distalen Ende (52A-D) erstreckt, zwischen 4 und 5 mm liegt.

11. Phakische Intraokularlinse (1) nach einem der vorstehenden Ansprüche, **dadurch gekennzeichnet, dass** ein Krümmungsradius (k) einer hinteren Oberfläche der Kuppel (K) zwischen 8 und 10 mm liegt.

12. Phakische Intraokularlinse (1) nach einem der vorstehenden Ansprüche, **dadurch gekennzeichnet, dass** eine hintere Oberfläche der Kuppel (K) glatt und konkav ist.

13. Phakische Intraokularlinse (1) nach einem der vorstehenden Ansprüche, **dadurch gekennzeichnet, dass** sie ein biegsames und biokompatibles Material umfasst, das zwischen 30 und 40 % Wasser umfasst, dessen Elastizitätsmodul kleiner als 1 GPa ist.

14. Phakische Intraokularlinse (1) nach einem der vorstehenden Ansprüche, **dadurch gekennzeichnet, dass** das zentrale optische Teil (2) eine durchgehende Bohrung (21) umfasst, die sich zwischen der vorderen (11) und der hinteren (12) Oberfläche erstreckt und eingerichtet ist, um einen Fluidstrom zu erlauben.

15. Phakische Intraokularlinse (1) nach einem der vorstehenden Ansprüche, **dadurch gekennzeichnet, dass** die Linse aus einer Licht brechenden oder beugenden Linse an erweiterter Tiefenschärfe besteht.

## Revendications

1. Implant phaque de chambre postérieure (1) comprenant :
- une surface antérieure (11) et une surface postérieure (12) ;
- une partie optique centrale (2) comprenant une lentille, et s'étendant radialement relativement à un axe optique (Z) dirigé de ladite surface antérieure (11) vers ladite surface postérieure (12) ;
- une partie haptique périphérique (3) s'étendant radialement vers l'extérieur relativement à ladite partie optique centrale (2) ;
- une pluralité d'haptiques flexibles (5A-D) allongées ; **caractérisé en ce que** :
- ladite partie haptique périphérique (3) comprend :
• une portion proximale (31) fixée à ladite partie optique centrale (2), et s'étendant de façon circonférentielle et symétrique autour de ladite partie optique centrale (2) ;
• une portion distale (32) fixée à ladite portion proximale (31) et comprenant une pluralité d'éléments de support (4A-D) s'étendant à la fois radialement vers l'extérieur et postérieurement relativement à ladite partie optique centrale (2) ;
lesdits éléments de support (4A-D) étant configurés pour supporter ledit implant phaque (1) sur une zonule ciliaire (97) lorsqu'il est en usage normal dans un œil (9) ;
ladite partie optique centrale (2) et ladite partie haptique périphérique (3) formant un dôme (K) ;
- chacune desdites haptiques flexibles (5A-D) s'étend à la fois de façon partiellement circonférentielle autour d'un desdits éléments de support (4A-D), et radialement vers l'extérieur relativement à ladite partie optique centrale (2) ;
un premier diamètre (81) dudit implant phaque (1) étant strictement plus grand qu'un deuxième diamètre (82) d'un ensemble optique consistant en ladite partie optique centrale (2) et ladite partie haptique périphérique (3) ; chacune desdites haptiques flexibles (5A-D) s'étendant au moins partiellement entre les premier (81) et deuxième (82) diamètres ;
chacune desdites haptiques flexibles (5A-D) comprenant :
• une extrémité proximale (51A-D) fixée à ladite portion proximale (31) de ladite partie haptique périphérique (3) ;
• une extrémité distale (52A-D) libre pour accrocher ledit implant phaque (1) dans un sulcus ciliaire (98) lorsqu'il est en usage normal dans un œil (9).

2. Implant phaque (1) selon la revendication précédente, **caractérisé en ce que** chacune desdites haptiques flexibles (5A-D) comprend une section intermédiaire (53A-D) comprise entre son extrémité proximale (51A-D) et son extrémité distale (52A-D), et apte à se courber lorsqu'une compression radiale et/ou axiale est exercée sur ladite chacune desdites haptiques flexibles (5A-D) ;
et **en ce que** :
- une section proximale de ladite chacune desdites haptiques flexibles (5A-D), s'étendant entre son extrémité proximale (51A-D) et sa section intermédiaire (53A-D), s'étend postérieurement relativement à ladite partie optique centrale (2) ;
- une section distale de ladite chacune desdites haptiques flexibles (5A-D), s'étendant entre sa section intermédiaire (53A-D) et son extrémité distale (52A-D), forme un angle (α) compris entre -15° et 15° avec une droite perpendiculaire (d) audit axe optique (Z).

3. Implant phaque (1) selon la revendication précédente, **caractérisé en ce que** chacune desdites haptiques flexibles (5A-D) est :
- d'une épaisseur (83), mesurée selon ledit axe optique (Z), décroissante de son extrémité proximale (51A-D) à son extrémité distale (52A-D) ;
- d'une largueur (84), mesurée perpendiculairement audit axe optique (Z) :
• décroissante de son extrémité proximale (51A-D) à sa section intermédiaire (53A-D) ;
• plus grande en son extrémité distale (52A-D) qu'en son extrémité proximale (51A-D).

4. Implant phaque (1) selon l'une quelconque des revendications précédentes, **caractérisé en ce que** ladite extrémité distale (52A-D) comprend des ondulations lisses (54, 55) agencées pour s'accrocher dans un sulcus ciliaire (98) lorsque ledit implant phaque (1) est en usage normal dans un œil (9).

5. Implant phaque (1) selon l'une quelconque des revendications précédentes, **caractérisé en ce que** chacune desdites haptiques flexibles (5A-D) comprend un renfoncement latéral (56A-D) dans son extrémité proximale (51A-D) apte à permettre à cette dernière d'opérer comme charnière entre ladite chacune desdites haptiques flexibles (5A-D) et ladite portion proximale (31) de ladite partie haptique périphérique (3).

6. Implant phaque (1) selon l'une quelconque des revendications précédentes, **caractérisé en ce qu'**il comprend :
- un premier (4A), un deuxième (4B), un troisième (4C) et un quatrième (4D) éléments de support, s'inscrivant dans un cylindre allongé le long dudit axe optique (Z), centré en ladite partie optique centrale (2), et d'un diamètre égal audit deuxième diamètre (82) ; lesdits premier (4A) et deuxième (4B) éléments de support s'étendant de façon diamétralement opposés de part et d'autre dudit axe optique (Z) ;
lesdits troisième (4C) et quatrième (4D) éléments de support s'étendant de façon diamétralement opposés de part et d'autre dudit axe optique (Z) ;
- une première (5A), une deuxième (5B), une troisième (5C) et une quatrième (5D) haptiques flexibles, s'inscrivant dans un autre cylindre allongé le long dudit axe optique (Z), centré en ladite partie optique centrale (2), et d'un diamètre égal audit premier diamètre (81) ;
lesdites première (5A) et deuxième (5B) haptiques flexibles s'étendant de façon diamétralement opposés de part et d'autre dudit axe optique (Z) ;
lesdites troisième (5C) et quatrième (5D) haptiques flexibles s'étendant de façon diamétralement opposés de part et d'autre dudit axe optique (Z).

7. Implant phaque (1) selon la revendication précédente, **caractérisé en ce que** :
- lesdits premier (4A), deuxième (4B), troisième (4C) et quatrième (4D) éléments de support sont orientés radialement vers l'extérieur relativement à ladite partie optique centrale (2), symétriquement selon des diagonales (r1, r2) d'un premier rectangle plan (R) s'intersectant sur ledit axe optique (Z) ;
- les extrémités distales (52A-D) desdites première (5A), deuxième (5B), troisième (5C) et quatrième (5D) haptiques flexibles sont essentiellement disposées symétriquement à des positions définies par des sommets (s1, s2, s3, s4) d'un deuxième rectangle plan (S) de côtés parallèles à des côtés dudit premier rectangle plan (R) ;
- les extrémités proximales (51A-D) desdites première (5A), deuxième (5B), troisième (5C) et quatrième (5D) haptiques flexibles sont essentiellement disposées symétriquement à des positions définies par des sommets (t1, t2, t3, t4) d'un troisième rectangle plan (T) de côtés parallèles à des côtés dudit premier rectangle plan (R) ;
- lesdites première (5A), deuxième (5B), troisième (5C) et quatrième (5D) haptiques flexibles s'étendent de façon partiellement circonférentielle autour desdits premier (4A), deuxième (4B), troisième (4C) et quatrième (4D) éléments de support, respectivement.

8. Implant phaque (1) selon l'une quelconque des deux revendications précédentes, **caractérisé en ce que** :
- les extrémités proximales (51A, 51C) desdites première (5A) et troisième (5C) haptiques flexibles sont séparées par une distance (85) inférieure à 1 mm ;
- les extrémités proximales (51B, 51D) desdites deuxième (5B) et quatrième (5D) haptiques flexibles sont séparées par une distance (85) inférieure à 1 mm ;
- les extrémités proximales (51A-D) desdites première (5A), deuxième (5B), troisième (5C) et quatrième (5D) haptiques flexibles sont à une distance (86) inférieure à 2 mm de la partie optique centrale (2).

9. Implant phaque (1) selon l'une quelconque des revendications précédentes, **caractérisé en ce que** :
- ledit première diamètre (81) est compris entre 14 et 15 mm ;
- ledit deuxième diamètre (82) est compris entre 11 et 12 mm.

10. Implant phaque (1) selon l'une quelconque des revendications précédentes, **caractérisé en ce qu'**une longueur (87) de chacune desdites haptiques flexibles (5A-D), mesurée le long d'une courbe (C) s'étendant de son extrémité proximale (51A-D) à son extrémité distale (52A-D), est comprise entre 4 et 5 mm.

11. Implant phaque (1) selon l'une quelconque des revendications précédentes, **caractérisé en ce que** un rayon de courbure (k) d'une surface postérieure dudit dôme (K) est compris entre 8 et 10 mm.

12. Implant phaque (1) selon l'une quelconque des revendications précédentes, **caractérisé en ce que** une surface postérieure dudit dôme (K) est lisse et concave.

13. Implant phaque (1) selon l'une quelconque des revendications précédentes, **caractérisé en ce qu'**il comprend un matériau souple et biocompatible, comprenant entre 30 et 40% d'eau, et d'un module de Young inférieur à 1 GPa.

14. Implant phaque (1) selon l'une quelconque des revendications précédentes, **caractérisé en ce que** la partie optique centrale (2) comprend un trou traversant (21) s'étendant entre lesdites surfaces antérieure (11) et postérieures (12) et agencé pour accueillir un écoulement fluidique.

15. Implant phaque (1) selon l'une quelconque des revendications précédentes, **caractérisé en ce que** ladite lentille consiste en une lentille à profondeur de champ réfractive ou diffractive.
